# EUROPEAN PATENT SPECIFICATION

(11) **EP 2 119 447 B1**
(45) Date of publication and mention of the grant of the patent: **03.04.2013**
(21) Application number: 08711317.1
(22) Date of filing: 15.02.2008
(51) Int. Cl.: A61K 39/00, C07K 14/71

(54) **VACCINE THERAPY FOR CHOROIDAL NEOVASCULARIZATION**
IMPFTHERAPIE FÜR CHOROIDALE NEOVASKULARISATION
THÉRAPIE DE VACCINS DESTINÉE À LA NÉOVASCULARISATION CHOROÏDIENNE

(30) Priority: 16.02.2007 JP 2007036318
(43) Date of publication of application: 18.11.2009
(73) Proprietor: Oncotherapy Science, Inc., Kawasaki-shi Kanagawa 213-0012 (JP)
(72) Inventor: TAMAKI, Yasuhiro, Tokyo 113-8654 (JP); TAHARA, Hideaki, Tokyo 113-8654 (JP)
(74) Representative: Vossius & Partner
(86) International application number: PCT/JP2008/052487
(87) International publication number: WO 2008/099908

(56) References cited:
- EP-A1- 1 502 599
- EP-A1- 1 548 032
- WO-A1-2004/024766
- WO-A2-03/028643
- JP-A- 2006 501 145
- JP-A- 2006 511 475
- KINOSE FUMI ET AL: "Inhibition of retinal and choroidal neovascularization by a novel KDR kinase inhibitor" MOLECULAR VISION, MOLECULAR VISION, SN, ATLANTA, vol. 11, 1 January 2005 (2005-01-01), pages 366-373, XP002527045 ISSN: 1090-0535 [retrieved on 2005-05-27]
- WADA S ET AL: "Rationale for Antiangiogenic Cancer Therapy with Vaccination Using Epitope Peptides Derived from Human Vascular Endothelial Growth Factor Receptor 2" CANCER RESEARCH, AMERICAN ASSOCIATION FOR CANCER RESEARCH, BALTIMORE, MD., US LNKD- DOI:10.1158/0008-5472.CAN-04-3759, vol. 65, no. 11, 1 June 2005 (2005-06-01), pages 4939-4946, XP002348368 ISSN: 0008-5472
- MOCHIMARU HIROSHI ET AL: "Suppression of choroidal neovascularization by dendritic cell vaccination targeting VEGFR2" IOVS, vol. 48, no. 10, October 2007 (2007-10), pages 4795-4801, XP002576197 ISSN: 0146-0404
- ZIERHUT M ET AL: "Immunology of the skin and the eye" IMMUNOLOGY TODAY, ELSEVIER PUBLICATIONS, CAMBRIDGE, GB LNKD- DOI:10.1016/0167-5699(96)30056-X, vol. 17, no. 10, 1 October 1996 (1996-10-01), pages 448-450, XP004034702 ISSN: 0167-5699
- GERY I ET AL: "Autoimmunity in the eye and its regulation" CURRENT OPINION IN IMMUNOLOGY, ELSEVIER, OXFORD, GB LNKD- DOI:10.1016/0952-7915(94)90017-5, vol. 6, no. 6, 1 December 1994 (1994-12-01), pages 938-945, XP023942196 ISSN: 0952-7915 [retrieved on 1994-12-01]
- KONDO A ET AL: "PROMINENT ROLES OF SECONDARY ANCHOR RESIDUES IN PEPTIDE BINDING TO HLA-A24 HUMAN CLASS I MOLECULES", JOURNAL OF IMMUNOLOGY, AMERICAN ASSOCIATION OF IMMUNOLOGISTS, US, vol. 155, 1 January 1995 (1995-01-01), pages 4307-4312, XP002931009, ISSN: 0022-1767
- KUBO R T ET AL: "DEFINITION OF SPECIFIC PEPTIDE MOTIFS FOR FOUR MAJOR HLA-A ALLELES", JOURNAL OF IMMUNOLOGY, AMERICAN ASSOCIATION OF IMMUNOLOGISTS, US, vol. 152, no. 8, 1 January 1994 (1994-01-01), pages 3913-3924, XP002936662, ISSN: 0022-1767
- RAMMENSEE H-G ET AL: "MHC LIGANDS AND PEPTIDE MOTIFS: FIRST LISTING", IMMUNOGENETICS, SPRINGER VERLAG, BERLIN, DE, vol. 41, 1 January 1995 (1995-01-01), pages 178-228, XP002919644, ISSN: 0093-7711, DOI: DOI:10.1007/BF00172063
- FINE A M ET AL: "EARLIEST SYMPTOMS CAUSED BY NEOVASCULAR MEMBRANES IN THE MACULA", ARCHIVES OF OPHTHALMOLOGY, vol. 104, no. 4, 1986, pages 513-514, ISSN: 0003-9950
- STREILEIN J WAYNE: "Ocular immune privilege: The eye takes a dim but practical view of immunity and inflammation.", JOURNAL OF LEUKOCYTE BIOLOGY, vol. 74, no. 2, August 2003 (2003-08), pages 179-185, ISSN: 0741-5400
- WU L ET AL: 'Neovascularization, Choroidal', [Online] Retrieved from the Internet: <URL:http://emedicine.medscape.com/article/ 1190818-overview> [retrieved on 2010-09-04]

### SEQUENCE LISTING

<110> THE UNIVERSITY OF TOKYO
<120> Vaccine therapy for choroidal neovascular
<130> SEN-A0701P
<150> JP 2007-036318 <151> 2007-02-16
<160> 12
<170> PatentIn version 3.4
<210> 1
   <211> 9
   <212> PRT
   <213> Homo sapiens
<400> 1
   Ala Met Phe Phe Trp Leu Leu Leu Val
   1               5
<210> 2
   <211> 9
   <212> PRT
   <213> Homo sapiens
<400> 2
   Val Ile Ala Met Phe Phe Trp Leu Leu
   1               5
<210> 3
   <211> 9
   <212> PRT
   <213> Homo sapiens
<400> 3
   Ala Val Ile Ala Met Phe Phe Trp Leu
   1 5
<210> 4
   <211> 9
   <212> PRT
   <213> Homo sapiens
<400> 4
   Lys Leu Ile Glu Ile Gly Val Gln Thr
   1 5
<210> 5
   <211> 9
   <212> PRT
   <213> Homo sapiens
<400> 5
   Tyr Met Ile Ser Tyr Ala Gly Met Val
   1 5
<210> 6
   <211> 10
   <212> PRT
   <213> Homo sapiens
<400> 6
   Ile Gln Ser Asp Val Trp Ser Phe Gly Val
   1 5 10
<210> 7
   <211> 9
   <212> PRT
   <213> Homo sapiens
<400> 7
   Val Tyr Ser Ser Glu Glu Ala Glu Leu
   1 5
<210> 8
   <211> 9
   <212> PRT
   <213> Homo sapiens
<400> 8
   Gly Tyr Arg Ile Tyr Asp Val Val Leu
   1 5
<210> 9
   <211> 9
   <212> PRT
   <213> Homo sapiens
<400> 9
   Ser Tyr Met Ile Ser Tyr Ala Gly Met
   1 5
<210> 10
   <211> 9
   <212> PRT
   <213> Homo sapiens
<400> 10
   Arg Phe Val Pro Asp Gly Asn Arg Ile
   1 5
<210> 11
   <211> 9
   <212> PRT
   <213> Homo sapiens
<400> 11
   Lys Trp Glu Phe Pro Arg Asp Arg Leu
   1 5
<210> 12
   <211> 9
   <212> PRT
   <213> Homo sapiens
<400> 12
   Asp Phe Leu Thr Leu Glu His Leu Ile
   1 5

## Description

### Technical Field

The present invention relates to pharmaceutical agents for treatment and/or prevention of diseases caused by choroid neovascularization (neovascular maculopathy). The present disclosure also relates to inhibitory agents for neovascularization in the choroid.

### Background Art

Exudative age-related macular degeneration (AMD) caused by choroid neovascularization (CNV) is one of the major causes of severe visual impairment in developed countries. Evidence to date suggests that vascular endothelial growth factor (VEGF) plays a central role in the development of CNV. For example, it has been reported that CNV is suppressed by compounds that inhibit the production of VEGF or compounds that inhibit the signal transduction pathway of VEGF. Furthermore, it has also been reported that anti-VEGF antibodies show higher therapeutic efficacy compared to conventional therapeutic methods including photodynamic therapy. Therefore, in recent years, anti-VEGF agents have become a main option for drug therapy against CNV.

VEGF signaling is mediated by two types of receptor tyrosine kinases, *i.e.,* VEGFR-1 and VEGFR-2. The two receptors are expressed on the human CNV membrane and the laboratory mouse CNV membrane. However, the role of VEGFR-1 signal transduction pathway in CNV is still controversial. For example, one study reports that the inhibition of VEGFR-1 signaling by oral administration of an antibody, gene knockdown, or siRNA inhibits CNV. Another study reports that, in the eye, activation of VEGFR-1 by VEGF or placental growth factor 1 (PIGF1), which is a ligand of VEGFR-2, leads to activation of CNV via activation of VEGFR-2 by SPARC.

On the other hand, for VEGFR-2, the finding that activation of VEGFR-2 signaling promotes CNV growth is generally accepted. Thus, antiangiogenic approaches targeting VEGFR-2, such as systemic or local administration of anti-VEGFR-2 agents or VEGFR-2 antibodies, and intravitreal administration of siRNA, are expected to inhibit VEGFR-2 signaling and CNV growth.

However, the problem with currently available anti-VEGF agents is that they need to be injected repeatedly at 4- to 6-week intervals. Furthermore, there is a high risk of severe complications such as endophthalmitis and retinal detachment. Therefore, it is desirable to establish a novel therapeutic method that replaces currently used anti-VEGF agents.

A vaccine using a peptide derived from human VEGF receptor 2 is known to induce cytotoxic T-lymphocytes (CTL) that have potent cytotoxicity against VEGFR-2-expressing endothelial cells in tumor tissues (Patent Document 1). However, no vaccine that induces cytotoxic T-lymphocytes (CTL) in the choroid is known. Furthermore, there are many unclear aspects in the mechanism of neovascularization in the choroid and in other tissues.

A prior art document of the present invention is shown below.
[Patent Document 1] WO/2004/024766

### Disclosure of the Invention

The invention relates to the embodiments as defined in the claims

### [Problems to be Solved by the Invention]

The present invention was made in view of the above circumstances. An objective to be achieved by the present invention is to provide pharmaceutical agents that target VEGFR-2, which is known as one of the proteins involved in choroid neovascularization, for treatment and/or prevention of diseases caused by choroid neovascularization (neovascular maculopathy). Another objective of the present disclosure is to provide agents for inhibition of choroid neovascularization.

### [Means for Solving the Problems]

To achieve the above-mentioned objectives, the present inventors focused on VEGFR-2, which is known as one of the proteins involved in choroid neovascularization. Specifically, the present inventors administered a vaccine containing a VEGFR-2-derived peptide as an antigen to model mice (A2/Kb transgenic mice) expressing human HLA-A*0201, and tested the vaccine effect. As a result, the present inventors discovered that the vaccine containing the peptide as an antigen inhibits choroid neovascularization.

There have been many reports on the connection between VEGF and choroid neovascularization. However, there are many unclear aspects in the mechanism of neovascularization in the choroid and in other tissues, and the role of VEGFR-1 and VEGFR-2 in neovascularization is still unknown. Specifically, although there are reports on the application of VEGFR-1 and VEGFR-2 to vaccine therapy against tumors, no report has shown their application to vaccine therapy against choroid neovascularization. This indicates that those skilled in the art recognized that, while VEGFR-1- and VEGFR-2-derived peptide vaccines are known to inhibit non-choroid neovascularization, they do not necessarily show similar effects in the choroid. Under such circumstances, the present inventors selected VEGFR-2 from many candidates as a target, and carried out experiments. As a result, the present inventors discovered that a vaccine containing a VEGFR-2-derived peptide as an antigen can inhibit choroid neovascularization. The present inventors also discovered that the vaccine containing a VEGFR-2-derived peptide as an antigen is effective not only for patients with severe symptoms but also for patients of early stage cases with relatively good vision. Specifically, the present disclosure provides [1] to [36] below:
[1] a pharmaceutical agent for treatment and/or prevention of a disease caused by choroid neovascularization (neovascular maculopathy), which comprises at least one of the peptides of (a) to (c) below as an active ingredient:
   (a) a peptide comprising the amino acid sequence of any of SEQ ID NOs: 1 to 12;
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells; and
   (c) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[2] the pharmaceutical agent of [1], wherein the disease caused by choroid neovascularization (neovascular maculopathy) is selected from exudative age-related macular degeneration, myopic macular degeneration, angioid streaks, central exudative chorioretinopathy, various retinal pigment epitheliopathies, choroidal atrophy, choroideremia, and choroidal osteoma;
[3] a vaccine for treatment and/or prevention of a disease caused by choroid neovascularization (neovascular maculopathy), which comprises at least one of the peptides of (a) and (b) below as an active ingredient:
   (a) a peptide comprising the amino acid sequence of any of SEQ ID NOs: 1 to 6; and
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells.
[4] a vaccine for treatment and/or prevention of a disease caused by choroid neovascularization (neovascular maculopathy), which comprises at least one of the peptides of (a) and (b) below as an active ingredient:
   (a) a peptide comprising the amino acid sequence of any of SEQ ID NOs: 7 to 12; and
   (b) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[5] the vaccine of [3], which is administered to a subject whose HLA antigen is HLA-A02;
[6] the vaccine of [4], which is administered to a subject whose HLA antigen is HLA-A24;
[7] the vaccine of [5] or [6], wherein the disease caused by choroid neovascularization (neovascular maculopathy) is selected from exudative age-related macular degeneration, myopic macular degeneration, angioid streaks, central exudative chorioretinopathy, various retinal pigment epitheliopathies, choroidal atrophy, choroideremia, and choroidal osteoma;
[8] a pharmaceutical agent for inhibiting choroid neovascularization, which comprises at least one of the peptides of (a) to (c) below as an active ingredient:
   (a) a peptide comprising the amino acid sequence of any of SEQ ID NOs: 1 to 12;
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells; and
   (c) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[9] a vaccine for inhibiting choroid neovascularization, which comprises at least one of the peptides of (a) and (b) below as an active ingredient:
   (a) a peptide comprising the amino acid sequence of any of SEQ ID NOs: 1 to 6; and
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells;
[10] a vaccine for inhibiting choroid neovascularization, which comprises at least one of the peptides of (a) and (b) below as an active ingredient:
   (a) a peptide comprising the amino acid sequence of any of SEQ ID NOs: 7 to 12; and
   (b) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[11] the vaccine of [9], which is administered to a subject whose HLA antigen is HLA-A02;
[12] the vaccine of [10], which is administered to a subject whose HLA antigen is HLA-A24;
[13] a method for treating and/or preventing a disease caused by choroid neovascularization (neovascular maculopathy), which comprises the step of administering to a subject a pharmaceutical agent comprising at least one of the peptides of (a) to (c) below as an active ingredient:
   (a) a peptide comprising the amino acid sequence of any of SEQ ID NOs: 1 to 12;
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells; and
   (c) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[14] the method of [13], wherein the disease caused by choroid neovascularization (neovascular maculopathy) is selected from exudative age-related macular degeneration, myopic macular degeneration, angioid streaks, central exudative chorioretinopathy, various retinal pigment epitheliopathies, choroidal atrophy, choroideremia, and choroidal osteoma;
[15] a method for treating and/or preventing a disease caused by choroid neovascularization (neovascular maculopathy), which comprises the step of administering to a subject a vaccine comprising at least one of the peptides of (a) and (b) below as an active ingredient:
   (a) a peptide comprising the amino acid sequence of any of SEQ ID NOs: 1 to 6; and
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells;
[16] a method for treating and/or preventing a disease caused by choroid neovascularization (neovascular maculopathy), which comprises the step of administering to a subject a vaccine comprising at least one of the peptides of (a) and (b) below as an active ingredient:
   (a) a peptide comprising the amino acid sequence of any of SEQ ID NOs: 7 to 12; and
   (b) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[17] the method of [15], wherein the administration is performed on a subject whose HLA antigen is HLA-A02;
[18] the method of [16], wherein the administration is performed on a subject whose HLA antigen is HLA-A24;
[19] the method of [17] or [18], wherein the disease caused by choroid neovascularization (neovascular maculopathy) is selected from exudative age-related macular degeneration, myopic macular degeneration, angioid streaks, central exudative chorioretinopathy, various retinal pigment epitheliopathies, choroidal atrophy, choroideremia, and choroidal osteoma;
[20] a method for inhibiting choroid neovascularization, which comprises the step of administering to a subject a pharmaceutical agent comprising at least one of the peptides of (a) to (c) below as an active ingredient:
   (a) a peptide comprising the amino acid sequence of any of SEQ ID NOs: 1 to 12;
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells; and
   (c) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[21] a method for inhibiting choroid neovascularization, which comprises the step of administering to a subject a vaccine comprising at least one of the peptides of (a) and (b) below as an active ingredient:
   (a) a peptide comprising the amino acid sequence of any of SEQ ID NOs: 1 to 6; and
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells;
[22] a method for inhibiting choroid neovascularization, which comprises the step of administering to a subject a vaccine comprising at least one of the peptides of (a) and (b) below as an active ingredient:
   (a) a peptide comprising the amino acid sequence of any of SEQ ID NOs: 7 to 12; and
   (b) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[23] the method of [21], wherein the administration is performed on a subject whose HLA antigen is HLA-A02;
[24] the method of [22], wherein the administration is performed on a subject whose HLA antigen is HLA-A24;
[25] use of a peptide of any of (a) to (c) below in the production of a pharmaceutical agent for treatment and/or prevention of a disease caused by choroid neovascularization (neovascular maculopathy):
   (a) a peptide comprising the amino acid sequence of any of SEQ ID NOs: 1 to 12;
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells; and
   (c) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[26] the use of [25], wherein the disease caused by choroid neovascularization (neovascular maculopathy) is selected from exudative age-related macular degeneration, myopic macular degeneration, angioid streaks, central exudative chorioretinopathy, various retinal pigment epitheliopathies, choroidal atrophy, choroideremia, and choroidal osteoma;
[27] use of a peptide of (a) or (b) below in the production of a vaccine for treatment and/or prevention of a disease caused by choroid neovascularization (neovascular maculopathy).
   (a) a peptide comprising the amino acid sequence of any of SEQ ID NOs: 1 to 6; and
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells;
[28] use of a peptide of (a) or (b) below in the production of a vaccine for treatment and/or prevention of a disease caused by choroid neovascularization (neovascular maculopathy):
   (a) a peptide comprising the amino acid sequence of any of SEQ ID NOs: 7 to 12; and
   (b) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[29] the use of [27], wherein the vaccine is intended for administration to a subject whose HLA antigen is HLA-A02;
[30] the use of [28], wherein the vaccine is intended for administration to a subject whose HLA antigen is HLA-A24;
[31] the use of [29] or [30], wherein the disease caused by choroid neovascularization (neovascular maculopathy) is selected from exudative age-related macular degeneration, myopic macular degeneration, angioid streaks, central exudative chorioretinopathy, various retinal pigment epitheliopathies, choroidal atrophy, choroideremia, and choroidal osteoma;
[32] use of a peptide of any of (a) to (c) below in the production of a pharmaceutical agent for inhibition of choroid neovascularization:
   (a) a peptide comprising the amino acid sequence of any of SEQ ID NOs: 1 to 12;
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells; and
   (c) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[33] use of a peptide of (a) or (b) below in the production of a vaccine for inhibition of choroid neovascularization:
   (a) a peptide comprising the amino acid sequence of any of SEQ ID NOs: 1 to 6; and
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells;
[34] use of a peptide of (a) or (b) below in the production of a vaccine for inhibition of choroid neovascularization:
   (a) a peptide comprising the amino acid sequence of any of SEQ ID NOs: 7 to 12; and
   (b) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[35] the use of [33], wherein the vaccine is intended for administration to a subject whose HLA antigen is HLA-A02;
[36] the use of [34], wherein the vaccine is intended for administration to a subject whose HLA antigen is HLA-A24;
[37] a peptide of any of (a) to (c) below for use in the treatment and/or prevention of a disease caused by choroid neovascularization (neovascular maculopathy):
   (a) a peptide comprising the amino acid sequence of any of SEQ ID NOs: 1 to 12;
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells; and
   (c) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[38] the peptide of [37], wherein the disease caused by choroid neovascularization (neovascular maculopathy) is selected from exudative age-related macular degeneration, myopic macular degeneration, angioid streaks, central exudative chorioretinopathy, various retinal pigment epitheliopathies, choroidal atrophy, choroideremia, and choroidal osteoma;
[39] a peptide of (a) or (b) below for use in the treatment and/or prevention of a disease caused by choroid neovascularization (neovascular maculopathy):
   (a) a peptide comprising the amino acid sequence of any of SEQ ID NOs: 1 to 6; and
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells;
[40] a peptide of (a) or (b) below for use in the treatment and/or prevention of a disease caused by choroid neovascularization (neovascular maculopathy):
   (a) a peptide comprising the amino acid sequence of any of SEQ ID NOs: 7 to 12; and
   (b) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[41] the peptide of [39], wherein the vaccine is intended for administration to a subject whose HLA antigen is HLA-A02;
[42] the peptide of [40], wherein the vaccine is intended for administration to a subject whose HLA antigen is HLA-A24;
[43] the peptide of [41] or [42], wherein the disease caused by choroid neovascularization (neovascular maculopathy) is selected from exudative age-related macular degeneration, myopic macular degeneration, angioid streaks, central exudative chorioretinopathy, various retinal pigment epitheliopathies, choroidal atrophy, choroideremia, and choroidal osteoma;
[44] a peptide of any of (a) to (c) below for use in the inhibition of choroid neovascularization:
   (a) a peptide comprising the amino acid sequence of any of SEQ ID NOs: 1 to 12;
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells; and
   (c) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[45] a peptide of (a) or (b) below for use in the inhibition of choroid neovascularization:
   (a) a peptide comprising the amino acid sequence of any of SEQ ID NOs: 1 to 6; and
   (b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells;
[46] a peptide of (a) or (b) below for use in the inhibition of choroid neovascularization:
   (a) a peptide comprising the amino acid sequence of any of SEQ ID NOs: 7 to 12; and
   (b) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells;
[47] the peptide of [45], wherein the vaccine is intended for administration to a subject whose HLA antigen is HLA-A02; and
[48] the peptide of [46], wherein the vaccine is intended for administration to a subject whose HLA antigen is HLA-A24.

### Brief Description of the Drawings

Fig. 1 presents a graph showing the fluorescent leakage index of choroidal neovessels assessed by fluorescent fundus angiography of the PBS administration group, IFA single administration group, and peptide vaccination group on the seventh day.
Fig. 2 presents photographs showing an example of fluorescent fundus angiography of the PBS administration group, IFA single administration group, and peptide vaccination group on the seventh day.
Fig. 3 presents a graph showing the choroidal neovessel cross-sectional area assessed by fluorescent fundus angiography of the PBS administration group, IFA single administration group, and peptide vaccination group on the seventh day.
Fig. 4 presents photographs showing an example of extended choroidal preparation of the PBS administration group, IFA single administration group, and peptide vaccination group on the seventh day.

### Mode for Carrying Out the Invention

The present invention relates to pharmaceutical agents for treatment and/or prevention of diseases caused by choroid neovascularization (neovascular maculopathy), which comprise a VEGFR-2-derived peptide as an active ingredient. Hereinafter, these pharmaceutical agents may be referred to as "pharmaceutical agents of the present invention". As described above, the present invention is based on the present inventors' finding that vaccines containing a VEGFR-2-derived peptide as an antigen are effective for inhibition of choroid neovascularization.

The amino acid sequence of human VEGFR-2 is known, and is described in, for example, U.S. Patent No. 5,861,301. Those skilled in the art having access to the information described in this patent document can easily obtain human VEGFR-2.

A VEGFR-2-derived peptide contained in the pharmaceutical agents of the present disclosure is preferably a nonamer or decamer partial peptide. More specifically, such peptides include:
(a) a peptide comprising the amino acid sequence of any of SEQ ID NOs: 1 to 12.
   There is no limitation on the peptides of the present disclosure, as long as the peptides have an activity of inducing CTL against VEGFR-2-expressing cells. More specifically, the peptides of the present disclosure also include:
(b) a peptide with one or more amino acid substitutions, deletions, additions, and/or insertions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells. Furthermore, one or two amino acids may be added to the N terminus and/or the C terminus of the peptides.
   Furthermore, the peptides of the present disclosure also include:
(c) a peptide with one or more amino acid substitutions, deletions, additions, and/or insertions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells.

The amino acid residues to be mutated are preferably mutated to other amino acids in which the properties of the amino acid side chains are maintained. Examples of amino acid side chain properties include: hydrophobic amino acids (A, I, L, M, F, P, W, Y, and V); hydrophilic amino acids (R, D, N, C, E, Q, G, H, K, S, and T); amino acids having aliphatic side chains (G, A, V, L, I, and P); amino acids having hydroxyl group-containing side chains (S, T, and Y); amino acids having sulfur atom-containing side chains (C and M); amino acids having carboxylic acid- and amide-containing side chains (D, N, E, and Q); amino acids having base-containing side chains (R, K, and H); and amino acids having aromatic ring-containing side chains (H, F, Y, and W) (all amino acids are represented by one-letter codes in parentheses). Amino acid substitution within each of these groups is called conservative substitution. A polypeptide having a modified amino acid sequence, in which one or more amino acid residues are deleted, added, and/or replaced with other amino acids in a certain amino acid sequence, is known to retain its original biological activity (Mark, D. F. et al., Proc. Natl. Acad. Sci. USA (1984) 81, 5662-6; Zoller, M. J. and Smith, M., Nucleic Acids Res. (1982) 10, 6487-500; Wang, A. et al., Science (1984) 224: 1431-3; Dalbadie-McFarland, G. et al., Proc. Natl. Acad. Sci. USA (1982) 79: 6409-13).

The number of mutated amino acids is not particularly limited. Generally, the number is seven or less, preferably five or less, and more preferably three or less (for example, two or less). The amino acid sequence identity can be determined, for example, using the BLAST algorithm by Karlin and Altschul (Proc. Natl. Acad. Sci. USA (1993) 90: 5873-7).

In the present disclosure, a peptide comprising the amino acid sequence of SEQ ID NO: 2 is particularly preferred.

Furthermore, a peptide of the present disclosure can be obtained by synthesizing the peptide from any position based on the amino acid sequence. Peptide synthesis can be performed according to methods conventionally used in peptide chemistry. Conventional synthesis methods are described in documents such as "Peptide Synthesis", Interscience, New York, 1966; "The Proteins", Vol. 2, Academic Press Inc., New York, 1976; "Peptide Synthesis (Peptide Gosei)", Maruzen, 1975; "Fundamentals and Experiments of Peptide Synthesis (Peptide Gosei no Kiso to Jikken)", Maruzen, 1985; and "The sequel of Development of Pharmaceuticals (Zoku Iyakuhin no Kaihatsu)", Vol. 14, Peptide Synthesis (Peptide Gosei), Hirokawa Shoten, 1991, and in publications such as International Publication No. WO99/67288.

Peptides of the present disclosure may also be synthesized by known genetic engineering methods. An example of the genetic engineering synthesis methods is the following method. A vector into which a DNA encoding a peptide of interest has been inserted is introduced into suitable host cells to produce transformed cells. The peptides of the present disclosure can be obtained by collecting the peptides produced in these transformed cells.

Furthermore, a peptide of the present disclosure can be produced initially as a fusion protein, which is then cleaved using an appropriate protease to obtain the peptide. In a method for preparing a fusion protein, a polynucleotide encoding a peptide of the present disclosure may be ligated in frame with a polynucleotide encoding another peptide, and this may be inserted into an expression vector for expression in a host. Techniques known to those skilled in the art can be used for this purpose. For peptides fused with the peptides of the present disclosure, one may use known peptides such as FLAG (Hopp, T. P. et al., BioTechnology (1988) 6, 1204-1210), 6x His consisting of six histidine (His) residues, 10x His, influenza hemagglutinin (HA), human c-myc fragments, VSV-GP fragments, p18HIV fragments, T7-tag, HSV-tag, E-tag, SV40T antigen fragments, lck tag, α-tubulin fragments, B-tag, and Protein C fragments. It is also possible to ligate the peptides of the present disclosure to glutathione-S-transferase (GST), influenza hemagglutinin (HA), immunoglobulin constant regions, β-galactosidase, maltose-binding protein (MBP), or such to produce the fusion proteins. The peptides of the present disclosure can be obtained by treating the fusion proteins produced in this manner with a suitable protease, and then colleting the peptides of interest. The peptides can be collected by methods known to those skilled in the art, such as affinity chromatography.

Furthermore, the peptides of the present disclosure have an activity of inducing cytotoxic i T cells (cell-mediated T-cell inducing activity) when the peptides are contacted *in vitro* or *in vivo* with cells having antigen-presenting ability. In particular, the peptides of the present disclosure preferably have high affinity to HLA antigens such as the A-02 and A-24 types, which are said to be frequently expressed in the Japanese population. HLA antigens such as the A-02 and A-24 types include subtypes such as A-0201 and A-2402, respectively. Examples of peptides having high binding affinity to HLA-A*0201 include the peptides of SEQ ID NOs: 1 to 6 (WO/2004/024766).

The peptides of the present disclosure; include not only peptides having high binding affinity to HLA-A*0201, but also peptides having high binding affinity to other types of HLA antigens (for example, HLA-A*2402). Among such peptides, examples of peptides having high binding affinity to HLA-A*2402 include the peptides of SEQ ID NOs: 7 to 12.

Once the vaccine effectiveness of a peptide derived from a certain protein is confirmed, those skilled in the art can easily search for other peptides that have high binding affinity to an HLA antigen from the same protein, and test the vaccine effect of the peptides. Furthermore, those skilled in the art can easily search for peptides that have high binding affinity to HLA antigens other than HLA-A*0201 from the same protein, and develop vaccines that can be applied to various racial groups. It is well known among those skilled in the art that, when a peptide derived from a certain protein activates immune reaction against the protein, a peptide derived from the same protein that shows high affinity to another HLA antigen also activates immune reaction against the protein, as long as the peptide has activity to induce CTL against the protein. More specifically, it is well known among those skilled in the art that when a peptide derived from VEGFR-2 (for example, SEQ ID NO: 2) inhibits neovascularization by activating immune reaction against VEGFR-2, a peptide derived from VEGFR-2 that shows high affinity to another HLA antigen (for example, a peptide that shows high affinity to HLA-A24) also inhibits neovascularization by activating immune reaction against VEGFR-2, as long as the peptide has activity to induce CTL against VEGFR-2.

Binding between a peptide of the present disclosure and an HLA antigen can be measured by isolating cells having an HLA antigen on the cell surface, such as dendritic cells, and evaluating the binding of the peptide to the cells using conventional methods.

Alternatively, software available on the internet, such as that described in Parker K. C., J. Immunol. 152, 163-75 (1994), can be used to calculate the binding affinity of various peptides towards HLA antigens *in silico.* The binding affinity towards HLA antigens can be measured according to methods described, for example, in Parker, K. C., J. Immunol., 152, 163-75 (1994), Nukaya, I., Int. J. Cancer, 80, 92-7 (1999), etc.

In the clinic, by determining in advance the type of HLA antigen carried by the patient in need of treatment, peptides that have high binding affinity to this antigen, or peptides that have high activity to induce cytotoxic T cells (CTL) via antigen presentation can be suitably selected. Peptides selected by such methods are also included in the peptides of the present disclosure.

In the present invention, "treatment" means amelioration of characteristic symptoms of diseases caused by choroid neovascularization (neovascular maculopathy) in patients who have actually developed the symptoms. In the present invention, there is no particular limitation on the degree of amelioration. Even if the degree is very low, it is included in the meaning of "treatment" of the present invention, as long as the symptoms can be ameliorated.

In the present invention, "prevention" means suppressing in advance the development of characteristic symptoms of diseases caused by choroid neovascularization (neovascular maculopathy). In the present invention, there is no limitation on the degree of suppression of the development. Even if the degree is very low, it is included in the meaning of "prevention" of the present invention, as long as the development can be suppressed.

Reduced vision is a symptom of diseases caused by choroid neovascularization (neovascular maculopathy). Determination of whether or not this symptom has been ameliorated can be assessed by vision test. Furthermore, the activity of choroidal neovessels can be evaluated by examination using fluorescent fundus angiography or optical coherence tomography devices.

The peptides of the present invention can be used alone for treatment and/or prevention. Alternatively, the peptides may be formulated by conventional formulation methods. In these cases, carriers, excipients, and such conventionally used for pharmaceuticals can be appropriately included in addition to the peptides of the present invention. For example, they may be used parenterally in the form of injectable sterile solutions or suspensions prepared with water or other pharmaceutically acceptable liquids. For example, they may be formulated by appropriately combining them with pharmaceutically acceptable carriers or media, more specifically, sterilized water or physiological saline solutions, vegetable oils, emulsifiers, suspending agents, surfactants, stabilizers, flavoring agents, excipients, vehicles, preservatives, binding agents, and such, and mixing them into a unit dosage form required for generally accepted pharmaceutical practice. The amount of active ingredient in the formulations is adjusted so that an appropriate dose within specified range is achieved.

The peptides of the present disclosure can be used as vaccines for inducing CTL *in vivo,* either singularly or as a combination of two or more types. More specifically, the present invention relates to vaccines for treatment and/or prevention of diseases caused by choroid neovascularization (neovascular maculopathy), which comprise a VEGFR-2-derived peptide as an active ingredient. By administering a peptide of the present disclosure, the peptide is displayed at a high density on the HLA antigen of antigen-presenting cells, and this induces CTL that reacts specifically to the displayed complex formed between the peptide and the HLA antigen, and the attack capability against vascular endothelial cells in the choroid is increased. Alternatively, dendritic cells are removed from a patient and stimulated with a peptide of the present invention to obtain antigen presenting cells that have the peptide of the present disclosure bound on the cell surface. By administering the cells to the patient, CTL is induced in the patient, and thus the attack capability against the target cells can be increased.

When using the peptides of the present disclosure as vaccines, they may be administered together with an adjuvant so that cellular immunity is effectively established, administered together with other active ingredients such as anticancer agents, or administered as particulate formulations. Adjuvants such as those described in the document, Johnson AG, Clin. Microbiol. Rev., 7:277-289, 1994, may be applied. Liposome preparations, particulate preparations produced by binding to few micrometer-diameter beads, lipid-bound preparations, and such may also be used.

Those skilled in the art can suitably plan the method, dosage, and period of administration of a vaccine of the present disclosure depending on the symptoms of the patient (subject) in need of administration of the vaccine of the present disclosure. The vaccines of the present disclosure can be administered both systemically and locally. Examples of systemic administration include oral administration, intradermal administration, subcutaneous administration, and intravenous injection. Examples of local administration include administration to the vicinity of the choroid.

The dosage may be, for example, 0.001 mg to 1000 mg, preferably 0.001 mg to 1000 mg, and more preferably 0.1 mg to 10 mg, but is not limited thereto. The vaccines are preferably administered once every few days or months, but the frequency is not limited thereto.

The pharmaceutical agents of the present invention are effective against diseases caused by choroid neovascularization (neovascular maculopathy). There is no limitation on the target diseases of the pharmaceutical agents of the present invention, as long as they are diseases caused by choroid neovascularization (neovascular maculopathy). Preferably, the diseases include neovascular maculopathy that accompany diseases such as exudative age-related macular degeneration, myopic macular degeneration, angioid streaks, central exudative chorioretinopathy, various retinal pigment epitheliopathies, choroidal atrophy, choroideremia, and choroidal osteoma. A particularly preferred example is exudative age-related macular degeneration.

The pharmaceutical agents of the present disclosure have a low risk of rapid visual reduction and development of severe complications post-treatment, which are problems in conventional therapeutic methods. Therefore, the pharmaceutical agents of the present disclosure can be applied not only to patients with severe symptoms but also to early-stage patients with relatively good vision. Since retinal damage is low in early-stage cases with relatively good vision, the visual prognosis post-treatment for advanced cases is expected to be much more favorable than in conventional treatment.

The present: disclosure based on the finding that vaccines containing a VEGFR-2-derived peptide as an antigen damage vascular endothelial cells by inducing cytotoxic T lymphocyte (CTL) activity. Therefore, the present disclosure provides agents for inhibiting choroid neovascularization, which comprise a VEGFR-2-derived peptide as an active ingredient. Specific descriptions of the VEGFR-2-derived peptide are mentioned above. In the present disclosure, there is no limitation on the degree of inhibition. Even if the degree is very low, it is included in the meaning of "inhibition" as long as neovascularization can be inhibited.

The inhibitory agents of the present disclosure can be used in the form of vaccines. More specifically, the present disclosure provides vaccines for inhibiting choroid neovascularization, which comprise a VEGFR-2-derived peptide as an active ingredient. Specific descriptions of the vaccines, e.g., sites, methods, and dosages of administration of the vaccines, are mentioned above.

### Examples

Herein below, the present invention will be specifically described with reference to the Examples, but it is not to be construed as being limited thereto.

### [Methods]

### Animals

C57b1/6 mice with body weight of 20 g to 25 g were obtained from CLEA Japan (Tokyo, Japan). All experiments were conducted in accordance with the Animal Care and Use Committee and the Statement for the Use of Animals in Ophthalmic and Vision Research by the Association for Research in Vision and Opthalmology. Mice used in the experiments express human HLA-A*0201.

### Antigenic Peptides

The mice were separated into three groups. PBS, an immunity adjuvant (incomplete Freund's adjuvant (IFA)), or a suspension of a human VEGF receptor 2-derived antigenic peptide and IFA was injected subcutaneously into the axillae twice (day 0 and day 10). The peptide consists of nine amino acids from position 773 of human VEGFR-2, VIAMFFWLL (SEQ ID NO: 2), which was confirmed to have antiangiogenic effect in a tumor model. Subsequently, on day 20, CNV was induced at three sites in both eyes using a semiconductor laser set to φ 200 µm, 200 mW, and 0.02 seconds.

### Experimental CNV

Experimental CNV was induced as described elsewhere with minor modifications. In short, general anesthesia was induced with an intraperitoneal injection (1 mL/kg) of a mixture (1:1) of ketamine hydrochloride (Ketalar^{®}, Sankyo, Tokyo, Japan) and xylazine hydrochloride (Celactal^{®}, Bayer, Tokyo, Japan). The pupil was dilated using one drop of 0.5% tropicamide (Mydrin^{®} M, Santen Pharmaceutical, Osaka, Japan) for photocoagulation. Six laser photocoagulations were applied to each eye between the major retinal vessels around the optic disc with a diode laser photocoagulator (DC-3000^{®}, NIDEK, Osaka, Japan) and a slit lamp delivery system (SL150, Topcon, Tokyo, Japan) at a spot size of 200 mm, duration of 0.02 seconds, and intensity of 200 mW.

### Evaluation_of the_Grade_of CNV by Fluorescein Angiography

Three and seven days after CNV induction (on day 23 and day 27), a total of 169 animals were used to examine the effect on leakage from the CNV lesions (on the third day, the number of animals from the control group, low-dose group, and high-dose group was 41, 34, and 18, respectively; on the seventh day, the number of animals from the control group, low-dose group, and high-dose group was 51,15, and 10, respectively). Masking the observers as to the administration, the lesions on the third day and seventh day were examined using the fluorescein fluorescent fundus angiography method as described elsewhere.

### Measurement of the CNV Size by Choroidal Flat Mount

A total of 36 mice were used for choroidal flat mount analysis (the number of mice from each group on the third day and the seventh day was 4 and 8, respectively). Three days and seven days after laser treatment, the size of CNV lesion was evaluated using choroidal flat mounts as previously described. The mice were anesthetized and perfused with 1 mL of phosphate buffered saline (PBS) containing 50 mg/mL FITC-conjugated dextran (4.4 kDa, 50 mg/kg body weight; Sigma). Then, the eyes were enucleated, and the cornea and lens were removed, and the entire retina was carefully dissected from the eye cup. Radial cuts were made from the edges of the equator, and the eyecup was flat-mounted on a glass slide with the sclera facing down. The flat-mounted sections were observed under a fluorescence microscope (Olympus DPSO), and the images were captured using a CCD camera. The data were imported into a computer using a software system (Studio lite). The outline of CNV region was assigned using a computer mouse, and the measurement was performed using a public image processing program by NIH. For statistical analysis, the lesion sizes were averaged to give one value for each individual. The data were expressed as mean ± standard error.

### Statistical Analysis

The fluorescein fluorescent fundus angiography scores were evaluated using Mann-Whitney U test. The size of CNV lesion was evaluated by ANOVA followed by Dunnett's post hoc test. Values of P < 0.05 were considered to be statistically significant for all forms of statistical analysis used.

### Ethical Considerations

While the inventors worked on decreasing the discomfort felt by the mice in accordance with the Manual for Animal Experiment Operation by the University of Tokyo, since a Freund's adjuvant was used for stronger immune activation, the level of suffering is Category C by the Scientists Center for Animal Welfare.

### [Results]

### Effect on CNV Leakage

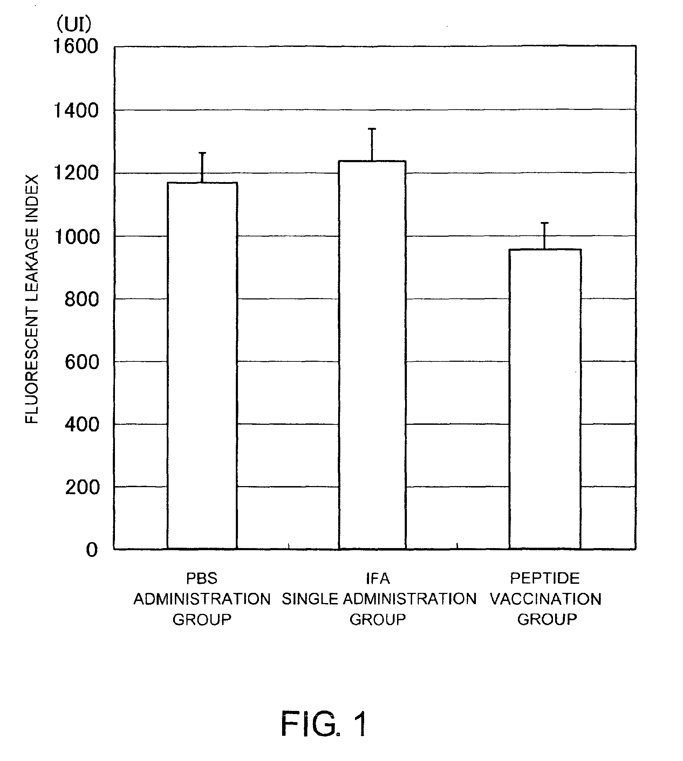

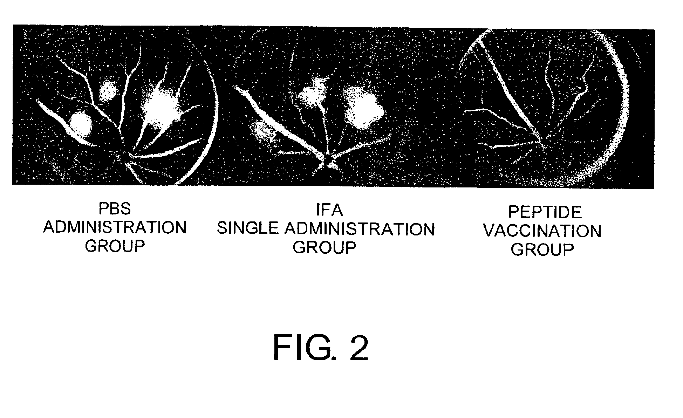

On the third day, no difference was observed among the control group, low-dose group, and high-dose group in the degree of fluorescein leakage. The fluorescein leakage from the CNV lesions in the non-administration group was increased on the seventh day compared to the third day. The fluorescein leakage from the CNV lesions was lower in the high-dose group than in the control (Figs. 1 and 2). Although it was not statistically significant, the leakage from CNV lesions was lower in animals of the low-dose group than in animals of the control group.

### Effect on CNV Lesion Size

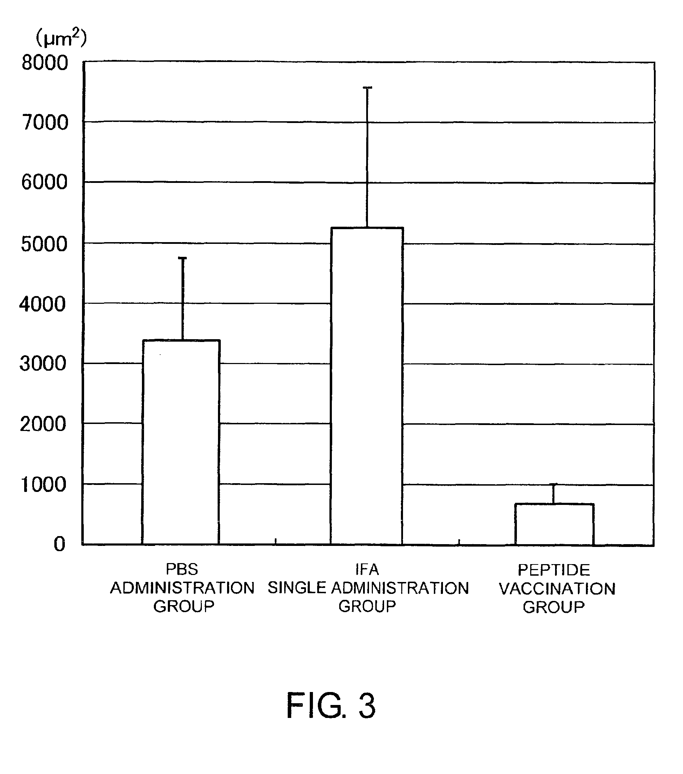

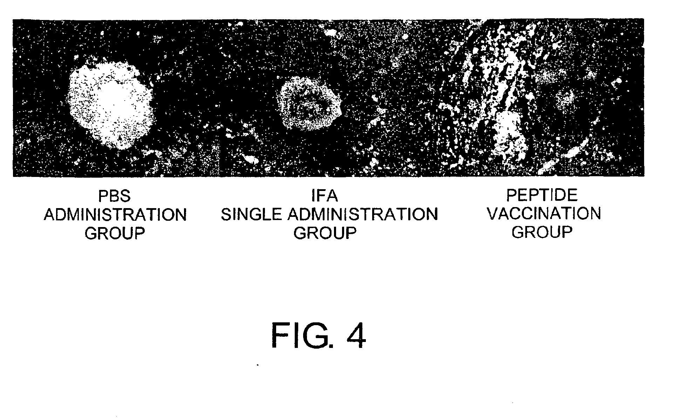

No difference was observed among the control group, low-dose group, and high-dose group in the CNV lesion size on the third day. The average CNV lesion size in the non-administration group was larger on the seventh day than on the third day. The CNV lesion size was significantly decreased in the low-dose group and the high-dose group compared to the control group (Figs. 3 and 4).

The present inventors showed that the peptide VIAMFFWLL(9) (SEQ ID NO: 2) suppresses VEGF up-regulation and inhibits CNV growth. VIAMFFWLL (SEQ ID NO: 2) is known to regulate immune responses. Since the activation of CNV is mediated by the inflammation process, and the inhibitory effect of VIAMFFWLL (SEQ ID NO: 2) on the *in vivo* CNV growth was observed only on the seventh day and not on the third day, it is likely that VIAMFFWLL (SEQ ID NO: 2) affects immune responses such as leukocyte infiltration, thereby regulating the neovascularization process, rather than inhibiting CNV via inhibition of neovascularization itself.

### Industrial Applicability

The present invention provides pharmaceutical agents (vaccines) for treatment and/or prevention of diseases caused by choroid neovascularization (neovascular maculopathy).

Conventionally, laser therapy, photodynamic therapy, operative therapy, drug therapy, and such have been performed as methods for treatment of diseases caused by choroid neovascularization (neovascular maculopathy). However, laser therapy could cause reduction of central vision. There are examples of rapid visual reduction following photodynamic therapy in cases with good vision. In operative therapy, there is a risk of postoperative complications associated with surgical invasion. In drug therapy, there is a risk of serious complications such as endophthalmitis and retinal detachment due to intraocular injection. That is, conventional therapies have a high risk of visual reduction due to treatment-associated adverse effects and complications. Therefore, it was difficult to treat early-stage cases with relatively good vision.

On the other hand, at the very least, the pharmaceutical agents (vaccines) of the present disclosure do not cause local complications in the eye, thus the above-mentioned risks are likely to be absent. Therefore, it is possible to treat early-stage cases with relatively good vision. Furthermore, since retinal damage is low in early-stage cases with relatively good vision, the visual prognosis post-treatment for advanced cases is expected to be much more favorable than in conventional treatment.

In the diseases of the present disclosure, the contralateral eye is known to have a high incidence of developing similar neovessels. However, there is no established method for prevention of neovessels in the contralateral eye. The vaccine therapy of the present disclosure can be expected to be effective for preventive treatment of the contralateral eye.

## Claims

1. A pharmaceutical agent for use in the treatment and/or prevention of a disease caused by choroid neovascularization (neovascular maculopathy), wherein the pharmaceutical agent comprises at least one of the peptides of (a) to (c) below as an active ingredient:
(a) a peptide comprising the amino acid sequence of any of SEQ ID NOs: 1 to 12;
(b) a peptide with one or two amino acid substitutions or additions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells; and
(c) a peptide with one or two amino acid substitutions or additions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells.

2. A vaccine selected from the group consisting of (i) and (ii) below:
(i) a vaccine for use in the treatment and/or prevention of a disease caused by choroid neovascularization (neovascular maculopathy), wherein the vaccine comprises at least one of the peptides of (a) and (b) below as an active ingredient:
(a) a peptide comprising the amino acid sequence of any of SEQ ID NOs: 1 to 6; and
(b) a peptide with one or two amino acid substitutions or additions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells;
(ii) a vaccine for use in the treatment and/or prevention of a disease caused by choroid neovascularization (neovascular maculopathy), wherein the vaccine comprises at least one of the peptides of (c) and (d) below as an active ingredient:
(c) a peptide comprising the amino acid sequence of any of SEQ ID NOs: 7 to 12; and
(d) a peptide with one or two amino acid substitutions or additions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells.

3. The vaccine of (i) of claim 2, which is intended for administration to a subject whose HLA antigen is HLA-A02.

4. The vaccine of (ii) of claim 2, which is intended for administration to a subject whose HLA antigen is HLA-A24.

5. The pharmaceutical agent of claim 1, or the vaccine of any one of claims 2 to 4, wherein the disease caused by choroid neovascularization (neovascular maculopathy) is selected from exudative age-related macular degeneration, myopic macular degeneration, angioid streaks, central exudative chorioretinopathy, various retinal pigment epitheliopathies, choroidal atrophy, choroideremia, and choroidal osteoma.

6. Use of a peptide of any of (a) to (d) below in the production of a pharmaceutical agent or vaccine for treatment and/or prevention of a disease caused by choroid neovascularization (neovascular maculopathy):
(a) a peptide comprising the amino acid sequence of any of SEQ ID NOs: 1 to 6;
(b) a peptide with one or two amino acid substitutions or additions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells;
(c) a peptide comprising the amino acid sequence of any of SEQ ID NOs: 7 to 12; and
(d) a peptide with one or two amino acid substitutions or additions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells.

7. The use of claim 6, wherein the peptide is (a) or (b), and wherein the pharmaceutical agent or vaccine is intended for administration to a subject whose HLA antigen is HLA-A02.

8. The use of claim 6, wherein the peptide is (c) or (d), and wherein the pharmaceutical agent or vaccine is intended for administration to a subject whose HLA antigen is HLA-A24.

9. The use of any one of claims 6 to 8, wherein the disease caused by choroid neovascularization (neovascular maculopathy) is selected from exudative age-related macular degeneration, myopic macular degeneration, angioid streaks, central exudative chorioretinopathy, various retinal pigment epitheliopathies, choroidal atrophy, choroideremia, and choroidal osteoma.

10. A peptide of any of (a) to (d) below for use in the treatment and/or prevention of a disease caused by choroid neovascularization (neovascular maculopathy):
(a) a peptide comprising the amino acid sequence of any of SEQ ID NOs: 1 to 6;
(b) a peptide with one or two amino acid substitutions or additions in the amino acid sequence of any of SEQ ID NOs: 1 to 6, wherein the second amino acid from the N terminus is leucine or methionine, and/or the C-terminal amino acid is valine or leucine, and wherein the peptide has an activity of inducing cytotoxic T cells;
(c) a peptide comprising the amino acid sequence of any of SEQ ID NOs: 7 to 12; and
(d) a peptide with one or two amino acid substitutions or additions in the amino acid sequence of any of SEQ ID NOs: 7 to 12, wherein the second amino acid from the N terminus is phenylalanine, tyrosine, methionine, or tryptophan, and/or the C-terminal amino acid is phenylalanine, leucine, isoleucine, tryptophan, or methionine, and wherein the peptide has an activity of inducing cytotoxic T cells.

11. The peptide of (a) or (b) of claim 10, wherein the peptide is intended for administration to a subject whose HLA antigen is HLA-A02.

12. The peptide of (c) or (d) of claim 10, wherein the peptide is intended for administration to a subject whose HLA antigen is HLA-A24.

13. The peptide of any one of claims 10 to 12, wherein the disease caused by choroid neovascularization (neovascular maculopathy) is selected from exudative age-related macular degeneration, myopic macular degeneration, angioid streaks, central exudative chorioretinopathy, various retinal pigment epitheliopathies, choroidal atrophy, choroideremia, and choroidal osteoma.

## Patentansprüche

1. Pharmazeutisches Mittel zur Verwendung in der Behandlung und/oder Prävention einer Krankheit, die durch Neovaskularisation der Choridea (neovaskuläre Makulopathie) verursacht wird, wobei das pharmazeutische Mittel mindestens eines der nachstehenden Peptide von (a) bis (c) als aktiven Bestandteil umfasst:
(a) ein Peptid, welches die Aminosäuresequenz nach einer der SEQ ID Nos: 1 bis 12 umfasst;
(b) ein Peptid mit ein oder zwei Aminosäuresubstitutionen oder -additionen in der Aminosäuresequenz nach einer der SEQ ID NOs: 1 bis 6, wobei die zweite Aminosäure vom N-Terminus ausgehend Leucin oder Methionin ist, und/oder die C-terminale Aminosäure Valin oder Leucin ist, und wobei das Peptid eine cytotoxische T-Zellen induzierende Aktivität aufweist; und
(c) ein Peptid mit ein oder zwei Aminosäuresubstitutionen oder -additionen in der Aminosäuresequenz nach einer der SEQ ID NOs: 7 bis 12, wobei die zweite Aminosäure vom N-Terminus ausgehend Phenylalanin, Tyrosin, Methionin oder Tryptophan ist, und/oder die C-terminale Aminosäure Phenylalanin, Leucin, Isoleucin, Tryptophan oder Methionin ist, und wobei das Peptid eine cytotoxische T-Zellen induzierende Aktivität aufweist.

2. Impfstoff, ausgewählt aus der Gruppe bestehend aus nachstehendem (i) und (ii):
(i) Impfstoff zur Verwendung in der Behandlung und/oder Prävention einer Krankheit, die durch Neovaskularisation der Choridea (neovaskuläre Makulopathie) verursacht wird, wobei der Impfstoff mindestens eines der nachstehenden Peptide von (a) und (b) als aktiven Bestandteil umfasst:
(a) ein Peptid, welches die Aminosäuresequenz nach einer der SEQ ID NOs: 1 bis 6 umfasst; und
(b) ein Peptid mit ein oder zwei Aminosäuresubstitutionen oder -additionen in der Aminosäuresequenz nach einer der SEQ ID NOs: 1 bis 6, wobei die zweite Aminosäure vom N-Terminus ausgehend Leucin oder Methionin ist, und/oder die C-terminale Aminosäure Valin oder Leucin ist, und wobei das Peptid eine cytotoxische T-Zellen induzierende Aktivität aufweist;
(ii) Impfstoff zur Verwendung in der Behandlung und/oder Prävention einer Krankheit, die durch Neovaskularisation der Choridea (Neovaskuläre Makulopathie) verursacht wird, wobei der Impfstoff mindestens eines der nachstehenden Peptide von (c) bis (d) als aktiven Bestandteil umfasst:
(c) ein Peptid, welches die Aminosäuresequenz nach einer der SEQ ID NOs: 7 bis 12 umfasst; und
(d) ein Peptid mit ein oder zwei Aminosäuresubstitutionen oder -additionen in der Aminosäuresequenz nach einer der SEQ ID NOs: 7 bis 12, wobei die zweite Aminosäure vom N-Terminus ausgehend Phenylalanin, Tyrosin, Methionin oder Tryptophan ist, und/oder die C-terminale Aminosäure Phenylalanin, Leucin, Isoleucin, Tryptophan oder Methionin ist, und wobei das Peptid eine cytotoxische T-Zellen induzierende Aktivität aufweist.

3. Impfstoff nach (i) aus Anspruch 2, welcher vorgesehen ist zur Verabreichung an ein Individuum, dessen HLA Antigen HLA-A02 ist.

4. Impfstoff nach (ii) aus Anspruch 2, welcher vorgesehen ist zur Verabreichung an ein Individuum, dessen HLA Antigen HLA-A24 ist.

5. Pharmazeutisches Mittel nach Anspruch 1 oder Impfstoff nach einem der Ansprüche 2 bis 4, wobei die durch Neovaskularisation der Choridea (neovaskuläre Makulopathie) verursachte Krankheit ausgewählt ist aus exsudativer, altersbezogener Makuladegeneration, myopischer Makuladegeneration, angioiden Netzhautstreifen, Chorioretinopathia centralis exsudativa, verschiedenen retinalen Pigmentepitheliopathien, choroidaler Atrophie, Choroideremia und choroidalem Osteom.

6. Verwendung eines Peptids nach einem der nachstehenden (a) bis (d) in der Produktion eines pharmazeutischen Mittels oder Impfstoffs zur Behandlung und/oder Prävention einer Krankheit, die durch Neovaskularisation der Choridea (neovaskuläre Makulopathie) verursacht wird:
(a) ein Peptid, welches die Aminosäuresequenz nach einer der SEQ ID NOs: 1 bis 6 umfasst;
(b) ein Peptid mit ein oder zwei Aminosäuresubstitutionen oder -additionen in der Aminosäuresequenz nach einer der SEQ ID NOs: 1 bis 6, wobei die zweite Aminosäure vom N-Terminus ausgehend Leucin oder Methionin ist, und/oder die C-terminale Aminosäure Valin oder Leucin ist, und wobei das Peptid eine cytotoxische T-Zellen induzierende Aktivität aufweist;
(c) ein Peptid, welches die Aminosäuresequenz nach einer der SEQ ID NOs: 7 bis 12 umfasst; und
(d) ein Peptid mit ein oder zwei Aminosäuresubstitutionen oder -additionen in der Aminosäuresequenz nach einer der SEQ ID NOs: 7 bis 12, wobei die zweite Aminosäure vom N-Terminus ausgehend Phenylalanin, Tyrosin, Methionin oder Tryptophan ist, und/oder die C-terminale Aminosäure Phenylalanin, Leucin, Isoleucin, Tryptophan oder Methionin ist, und wobei das Peptid eine cytotoxische T-Zellen induzierende Aktivität aufweist.

7. Verwendung nach Anspruch 6, wobei das Peptid (a) oder (b) ist und wobei das pharmazeutische Mittel oder der Impfstoff vorgesehen ist zur Verabreichung an ein Individuum, dessen HLA-Antigen HLA-A02 ist.

8. Verwendung nach Anspruch 6, wobei das Peptid (c) oder (d) ist und wobei das pharmazeutische Mittel oder der Impfstoff vorgesehen ist zur Verabreichung an ein Individuum, dessen HLA-Antigen HLA-A24 ist.

9. Verwendung nach einem der Ansprüche 6 bis 8, wobei die durch Neovaskularisation der Choridea (neovaskuläre Makulopathie) verursachte Krankheit ausgewählt ist aus exsudativer, altersbezogener Makuladegeneration, myopischer Makuladegeneration, angioiden Netzhautstreifen, Chorioretinopathia centralis exsudativa, verschiedenen retinalen Pigmentepitheliopathien, choroidaler Atrophie, Choroideremia und choroidalem Osteom.

10. Peptid nach einem der nachstehenden (a) bis (d) zur Verwendung in der Behandlung und/oder Prävention einer Krankheit, die durch Neovaskularisation der Choridea (neovaskuläre Makulopathie) verursacht wird:
(a) ein Peptid, welches die Aminosäuresequenz nach einer der SEQ ID NOs: 1 bis 6 umfasst;
(b) ein Peptid mit ein oder zwei Aminosäuresubstitutionen oder -additionen in der Aminosäuresequenz nach einer der SEQ ID Nos: 1 bis 6, wobei die zweite Aminosäure vom N-Terminus ausgehend Leucin oder Methionin ist, und/oder die C-terminale Aminosäure Valin oder Leucin ist, und wobei das Peptid eine cytotoxische T-Zellen induzierende Aktivität aufweist.;
(c) ein Peptid, welches die Aminosäuresequenz nach einer der SEQ ID NOs: 7 bis 12 umfasst; und
(d) ein Peptid mit ein oder zwei Aminosäuresubstitutionen oder -additionen in der Aminosäuresequenz nach einer der SEQ ID NOs: 7 bis 12, wobei die zweite Aminosäure vom N-Terminus ausgehend Phenylalanin, Tyrosin, Methionin oder Tryptophan ist, und/oder die C-terminale Aminosäure Phenylalanin, Leucin, Isoleucin, Tryptophan oder Methionin ist, und wobei das Peptid eine cytotoxische T-Zellen induzierende Aktivität aufweist.

11. Peptid nach (a) oder (b) gemäß Anspruch 10, wobei das Peptid vorgesehen ist zur Verabreichung an ein Individuum, dessen HLA-Antigen HLA-A02 ist.

12. Peptid nach (c) oder (d) gemäß Anspruch 10, **dadurch gekennzeichnet, dass** das Peptid vorgesehen ist zur Verabreichung an ein Individuum, dessen HLA-Antigen HLA-A24 ist.

13. Peptid nach einem der Ansprüche 10 bis 12, wobei die durch Neovaskularisation der Choridea (neovaskuläre Makulopathie) verursachte Krankheit ausgewählt ist aus exsudativer, altersbezogener Makuladegeneration, myopischer Makuladegeneration, angioiden Netzhautstreifen, Chorioretinopathia centralis exsudativa, verschiedenen retinalen Pigmentepitheliopathien, choroidaler Atrophie, Choroideremia und choroidalem Osteom.

## Revendications

1. Agent pharmaceutique pour l'utilisation dans le traitement et/ou la prévention d'une maladie provoquée par la néovascularisation de la choroïde (maculopathie néovasculaire), dans lequel l'agent pharmaceutique comprend au moins un des peptides de (a) à (c) ci-dessous comme principe actif :
(a) un peptide comprenant la séquence d'acides aminés de l'une quelconque des SEQ ID N°1 à 12 ;
(b) un peptide ayant une ou deux substitutions ou additions d'acides aminés dans la séquence d'acides aminés de l'une quelconque des SEQ ID N°1 à 6, dans lequel le deuxième acide aminé à partir de la terminaison N est la leucine ou la méthionine, et/ou l'acide aminé C-terminal est la valine ou la leucine, et dans lequel le peptide a une activité induisant les cellules T cytotoxiques ; et
(c) un peptide ayant une ou deux substitutions ou additions d'acides aminés dans la séquence d'acides aminés de l'une quelconque des SEQ ID N°7 à 12, dans lequel le deuxième acide aminé à partir de la terminaison N est la phénylalanine, la tyrosine, la méthionine, ou le tryptophane, et/ou l'acide aminé C-terminal est la phénylalanine, la leucine, l'isoleucine, le tryptophane, ou la méthionine, et dans lequel le peptide a une activité induisant les cellules T cytotoxiques.

2. Vaccin choisi dans le groupe constitué par (i) et (ii) ci-dessus
(i) un vaccin pour l'utilisation dans le traitement et/ou la prévention d'une maladie provoquée par la néovascularisation de la choroïde (maculopathie néovasculaire), dans lequel le vaccin comprend au moins un des peptides de (a) et (b) ci-dessous comme principe actif :
(a) un peptide comprenant la séquence d'acides aminés de l'une quelconque des SEQ ID N°1 à 6 ; et
(b) un peptide ayant une ou deux substitutions ou additions d'acides aminés dans la séquence d'acides aminés de l'une quelconque des SEQ ID N° 1 à 6, dans lequel le deuxième acide aminé à partir de la terminaison N est la leucine ou la méthionine, et/ou l'acide aminé C-terminal est la valine ou la leucine, et dans lequel le peptide a une activité induisant les cellules T cytotoxiques ;
(ii) un vaccin pour l'utilisation dans le traitement et/ou la prévention d'une maladie provoquée par la néovascularisation de la choroïde (maculopathie néovasculaire), dans lequel le vaccin comprend au moins un des peptides de (c) et (d) ci-dessous comme principe actif :
(c) un peptide comprenant la séquence d'acides aminés de l'une quelconque des SEQ ID N°7 à 12 ; et
(d) un peptide ayant une ou deux substitutions ou additions d'acides aminés dans la séquence d'acides aminés de l'une quelconque des SEQ ID N°7 à 12, dans lequel le deuxième acide aminé à partir de la terminaison N est la phénylalanine, la tyrosine, la méthionine, ou le tryptophane, et/ou l'acide aminé C-terminal est la phénylalanine, la leucine, l'isoleucine, le tryptophane, ou la méthionine, et dans lequel le peptide a une activité induisant les cellules T cytotoxiques.

3. Vaccin du (i) selon la revendication 2, qui est destiné à l'administration chez un sujet dont l'antigène HLA est le HLA-A02.

4. Vaccin du (ii) selon la revendication 2, qui est destiné à l'administration chez un sujet dont l'antigène HLA est le HLA-A24.

5. Agent pharmaceutique selon la revendication 1 ou vaccin selon l'une quelconque des revendications 2 à 4, dans lequel la maladie provoquée par la néovascularisation de la choroïde (maculopathie néovasculaire) est choisie parmi la dégénérescence maculaire exsudative liée à l'âge, la dégénérescence maculaire myopique, les stries angioïdes, la choriorétinopathie exsudative centrale, les diverses épithéliopathies pigmentaires rétiniennes, l'atrophie choroïdienne, la choroïdérémie, et l'ostéome choroïdien.

6. Utilisation d'un peptide selon l'un quelconque des (a) à (d) ci-dessous dans la production d'un agent pharmaceutique ou d'un vaccin pour le traitement et/ou la prévention d'une maladie provoquée par la néovascularisation de la choroïde (maculopathie néovasculaire) :
(a) un peptide comprenant la séquence d'acides aminés de l'une quelconque des SEQ ID N°1 à 6 ;
(b) un peptide ayant une ou deux substitutions ou additions d'acides aminés dans la séquence d'acides aminés de l'une quelconque des SEQ ID N°1 à 6, dans lequel le deuxième acide aminé à partir de la terminaison N est la leucine ou la méthionine, et/ou l'acide aminé C-terminal est la valine ou la leucine, et dans lequel le peptide a une activité induisant les cellules T cytotoxiques ;
(c) un peptide comprenant la séquence d'acides aminés de l'une quelconque des SEQ ID N°7 à 12 ; et
(d) un peptide ayant une ou deux substitutions ou additions d'acides aminés dans la séquence d'acides aminés de l'une quelconque des SEQ ID N°7 à 12, dans lequel le deuxième acide aminé à partir de la terminaison N est la phénylalanine, la tyrosine, la méthionine, ou le tryptophane, et/ou l'acide aminé C-terminal est la phénylalanine, la leucine, l'isoleucine, le tryptophane, ou la méthionine, et dans lequel le peptide a une activité induisant les cellules T cytotoxiques.

7. Utilisation selon la revendication 6, dans laquelle le peptide est (a) ou (b), et dans laquelle l'agent pharmaceutique ou le vaccin est destiné à l'administration chez un sujet dont l'antigène HLA est le HLA-A02.

8. Utilisation selon la revendication 6, dans laquelle le peptide est (c) ou (d), et dans laquelle l'agent pharmaceutique ou le vaccin est destiné à l'administration chez un sujet dont l'antigène HLA est le HLA-A24.

9. Utilisation selon l'une quelconque des revendications 6 à 8, dans laquelle la maladie provoquée par la néovascularisation de la choroïde (maculopathie néovasculaire) est choisie parmi la dégénérescence maculaire exsudative liée à l'âge, la dégénérescence maculaire myopique, les stries angioïdes, la choriorétinopathie exsudative centrale, les diverses épithéliopathies pigmentaires rétiniennes, l'atrophie choroïdienne, la choroïdérémie, et l'ostéome choroïdien.

10. Peptide selon l'un quelconque des (a) à (d) ci-dessous pour l'utilisation dans le traitement et/ou la prévention d'une maladie provoquée par la néovascularisation de la choroïde (maculopathie néovasculaire) :
(a) un peptide comprenant la séquence d'acides aminés de l'une quelconque des SEQ ID N°1 à 6 ;
(b) un peptide ayant une ou deux substitutions ou additions d'acides aminés dans la séquence d'acides aminés de l'une quelconque des SEQ ID N°1 à 6, dans lequel le deuxième acide aminé à partir de la terminaison N est la leucine ou la méthionine, et/ou l'acide aminé C-terminal est la valine ou la leucine, et dans lequel le peptide a une activité induisant les cellules T cytotoxiques ;
(c) un peptide comprenant la séquence d'acides aminés de l'une quelconque des SEQ ID N°7 à 12 ; et
(d) un peptide ayant une ou deux substitutions ou additions d'acides aminés dans la séquence d'acides aminés de l'une quelconque des SEQ ID N°7 à 12, dans lequel le deuxième acide aminé à partir de la terminaison N est la phénylalanine, la tyrosine, la méthionine, ou le tryptophane, et/ou l'acide aminé C-terminal est la phénylalanine, la leucine, l'isoleucine, le tryptophane, ou la méthionine, et dans lequel le peptide a une activité induisant les cellules T cytotoxiques.

11. Peptide du (a) ou (b) selon la revendication 10, dans lequel le peptide est destiné à l'administration chez un sujet dont l'antigène HLA est le HLA-A02.

12. Peptide du (c) ou (d) selon la revendication 10, dans lequel le peptide est destiné à l'administration chez un sujet dont l'antigène HLA est le HLA-A24.

13. Peptide selon l'une quelconque des revendications 10 à 12, dans lequel la maladie provoquée par la néovascularisation de la choroïde (maculopathie néovasculaire) est choisie parmi la dégénérescence maculaire exsudative liée à l'âge, la dégénérescence maculaire myopique, les stries angioïdes, la choriorétinopathie exsudative centrale, les diverses épithéliopathies pigmentaires rétiniennes, l'atrophie choroïdienne, la choroïdérémie, et l'ostéome choroïdien.
